# EUROPEAN PATENT APPLICATION

(11) **EP 4 012 015 A1**
(43) Date of publication of application: **15.06.2022**
(21) Application number: 20213856.6
(22) Date of filing: 14.12.2020
(51) Int. Cl.: C12M 1/34, C12M 1/00, C12P 7/10, C12N 1/36

(54) **METHOD AND SYSTEM FOR CONTROL OF A MICROBIAL FERMENTATION PROCESS**

(71) Applicant: Sekab E-Technology AB, 891 26 Örnsköldsvik (SE)
(72) Inventor: SIBBESEN, Ole, 2880 Bagsvaerd (DK); CAVKA, Adnan, 891 96 Arnäsvall (SE)
(74) Representative: Kransell & Wennborg KB

(57) **Abstract**

The present disclosure generally relates to a method for control of a microbial fermentation process involving fermentation of sugars from lignocellulosic biomass to fermentation products. The method comprising the steps of: providing a fermentation media comprising at least xylose and glucose; fermenting the fermentation media by means of fermentation microorganisms of yeast; and intentionally increasing the concentration of ethanol present during the fermentation to an elevated ethanol concentration by the addition of ethanol or an ethanol-producing substance.

## Description

### TECHNICAL FIELD

The present disclosure generally relates to a method and system for control of a microbial fermentation process involving fermentation of sugars from lignocellulosic biomass to fermentation products.

### BACKGROUND

Lignocellulosic residues from forestry are attractive as feedstocks for the production of green chemicals and fuels, since they are abundant, relatively inexpensive, and not used for food. Lignocellulose consists mainly of lignin and two classes of polysaccharides, cellulose and hemicellulose. Cellulose is composed of polysaccharide chains of several hundred to over ten thousand linked glucose units, whereas hemicellulose is a polysaccharide composed of xylose, other pentose sugars and various hexose sugars, e.g. glucose and mannose. In lignocellulose, cellulose and hemicellulose are tightly associated to lignin, a polyphenolic compound that ties the cellulose and hemicellulose polymers together, thus providing the lignocellulose with rigidity and mechanical strength.

For example, in the production of ethanol from lignocellulosic materials, various pretreatment and hydrolysis steps are typically used to separate cellulose, hemicellulose and lignin and subsequently to degrade the cellulose and hemicellulose polysaccharides to fermentable saccharides in a fermentation media, often referred to as "hydrolysate". The fermentable saccharides are then converted to ethanol by means of fermenting microorganisms, such as yeast or bacteria, and the ethanol is recovered by means of distillation. The yeast Saccharomyces cerevisiae, for example, metabolizes hexose sugars and is a microorganism suitable for industrial processes for cellulosic bioethanol production. However, Saccharomyces cerevisiae does not naturally ferment xylose that comprises a major part of many types of lignocellulosic material, why much attention has been put forth to engineering yeast to also acquire the ability to ferment xylose to ethanol and therefore to more fully utilize the available sugars from lignocellulose.

However, even with engineered yeast being able to ferment xylose, the fermenting rate of xylose is significantly slower than that of glucose. In addition, the fermentation rate of xylose has been found to correlate to the glucose concentration. It seems that the xylose is taken up by the same transmembrane transporters as glucose, but that the transmembrane transporters have a lower affinity towards xylose than towards glucose. Additionally, synthesis of the transmembrane transporters is induced by glucose but not by xylose, meaning that the amount of transmembrane transporters gets much lower when glucose has been exhausted and xylose is the only remaining fermentable sugar.

While the above improvements of xylose fermentation have led to an increased yield of ethanol based on sugars from lignocellulose, there are still potential for improvements related to the ability and rate of xylose fermentation.

### SUMMARY

In view of the above, it is an object of the present invention to provide improvements with respect to methods and arrangements for fermentation of lignocellulosic biomass, particularly to achieve a more favourable process for the fermentation of xylose

According to a first aspect of the present invention, a method for control of a microbial fermentation process involving fermentation of sugars from lignocellulosic biomass to fermentation products is provided. The method comprising the steps of:
- providing a fermentation media comprising at least xylose and glucose;
- fermenting the fermentation media by means of fermentation microorganisms of yeast; and
- intentionally increasing the concentration of ethanol present during the fermentation to an elevated ethanol concentration by the addition of ethanol or an ethanol-producing substance.

Hereby, the fermentation of xylose relative the fermentation of glucose is increased. In other words, the relative fermentation rate of xylose to the fermentation rate of glucose is increased. The inventors have realized that the elevated ethanol concentration results in that the yeast ferment the xylose and glucose more equally compared to normal conditions, or conditions in which the ethanol concentration is not intentionally increased to the elevated ethanol concentration. Thus, the elevated ethanol concentration delays the glucose fermentation compared to that of xylose, so that more xylose can be fermented while glucose is still present to induce transmembrane transporters. Without being bound by any theory, by fermenting xylose in the presence of glucose at the elevated ethanol concentration, transmembrane transporter affinity towards glucose is reduced. For example, this is advantageous as instability issues related to the decrease in xylose fermentation of the engineered yeast related to the loss of xylose fermenting capability, in which the glucose-only fermenting yeasts outgrows glucose/xylose-fermenting yeasts, can be reduced or even avoided. This is further described later in the text.

It should be understood that the ethanol producing substance is a substance that will be converted to ethanol in situ.

According to at least one example embodiment, the elevated ethanol concentration is adapted such that the fermentation rate of xylose is above 20 % the fermentation rate of glucose, such as e.g. above 40 %, or above 60 % or above 80 % the fermentation rate of glucose.

Hereby, the elevated ethanol concentration may be adapted to correspond to the desired relation or ratio of the fermentation rate of xylose to the fermentation rate of glucose. For example, the elevated ethanol concentration is adapted such that the fermentation rate of xylose is equal to, or substantial equal to, the fermentation rate of glucose. Thus, fermentation xylose and glucose can be carried out at an equal, or substantially equal fermentation rate. The inventors believe, again not by being bound by any theory, that the elevated ethanol concentration makes it possible to keep glucose present longer and thus the transmembrane transporters in the yeast induced until xylose is exhausted (i.e. all xylose is fermented). According to one example embodiment, the elevated ethanol concentration is adapted such that the fermentation rate of xylose is within 70 % - 100 % of the fermentation rate of glucose.

According to at least one example embodiment, the term "intentionally elevated ethanol concentration" is simply referred to as an "elevated ethanol concentration". The intentionally elevated ethanol concentration is referring to a the ethanol concentration, which at least during a portion of the fermentation process, that is higher than the ethanol concentration normally obtained during a corresponding portion of the fermentation process, under the same conditions and for the same input values, except for the means causing the elevated ethanol concentration. The elevated ethanol concentration may be reached by achieving a surplus ethanol concentration obtained by e.g. direct addition to an ongoing fermentation process, or to a feed to be subsequently used in a fermentation process, of ethanol, or of an ethanol containing fluid having a higher ethanol content than the broth in the fermentation system without such additions, causing the broth being processed to have a higher ethanol concentration during the remainder of the fermentation process than without this addition. The elevated ethanol concentration may alternatively be reached by direct addition to an ongoing fermentation process, or to a feed subsequently to be used in a fermentation process, of a compound that is converted to ethanol "in situ" causing the broth being processed in the fermentation system to for at least some of the remainder part of the fermentation process to contain a higher ethanol content, than the broth otherwise would have at the corresponding part of the fermentation without this addition.

According to at least one example embodiment, the step of fermenting comprises co-fermenting the xylose and glucose.

Thus, the elevated ethanol concentration balances the co-fermenting process of xylose and glucose, as the presence of glucose during the co-fermentation keep the transmembrane transporters induced until both xylose and glucose have been exhausted, effectively enabling a faster xylose fermentation at least through a part of the fermentation process.

Additionally or alternatively, the step of fermenting comprises at least partly sequentially fermenting the xylose and glucose, for example by utilizing a plurality of fermentation units as will be described later in the text. Thus, at least a part of the fermenting comprises fermenting solely the xylose, and/or solely the glucose (possibly together with another hexose, such as mannose). According to at least one example embodiment, the fermenting comprises the two sub-steps of fermenting solely the xylose and co-fermenting the xylose and glucose in a preferred order, and/or comprises the two sub-steps of fermenting solely the glucose and co-fermenting the xylose and glucose in a preferred order, wherein at least one of the sub-steps are performed at the elevated ethanol concentration. Moreover, at least one the sub-steps may be performed at an untampered ethanol concentration, or at least at an ethanol concentration which is not intentionally increased to the elevated ethanol concentration.

According to at least one example embodiment, the method further comprises the steps of:
- determining the ethanol concentration during the step of fermenting,
- in response of determining that the concentration of ethanol is below a threshold limit, adjusting the ethanol concentration to be above the threshold limit.

Hereby, an efficient means for adjusting the concentration of ethanol is provided, so that the elevated ethanol concentration is maintained during fermenting. For example, the ethanol concentration is determined directly during the step of fermenting, e.g. based on an online measurement of the fermenting slurry comprising the fermenting media, the yeast, and any produced fermented product. Hereby, the delay between determining the ethanol concentration and adjusting the ethanol concentration in response to the determination, is minimized. Alternatively, the ethanol concentration is determined in an off-gas from the fermenting slurry.

According to at least one example embodiment, the threshold limit is based on the relation or ratio of fermentation rates of glucose and xylose.

Such threshold limit is beneficial to use, as at least one purpose of the elevated ethanol concentration is to reduce the difference in fermentation rates between glucose and xylose. Thus, for example, the threshold limit of the elevated ethanol concentration is set based on that the fermentation rate of xylose is at least above 20 %, or above 40 %, or above 60 % or above 80 % the fermentation rate of glucose. The threshold limit may furthermore be based on fermentation parameters such as e.g. amount of fermentable sugars present in the media before being fermented or partially fermented.

According to at least one example embodiment, the step of fermenting is carried out in at least two separate fermentation units, wherein the concentration of ethanol is intentionally increased to the elevated ethanol concentration in at least one of the fermentation units.

Hereby, the step of fermenting can be divided between different fermentation units. For example, the concentration of ethanol may be intentionally increased to the elevated ethanol concentration in at least two fermentation units.

According to at least one example embodiment, the step of fermenting is carried out in at least two separate fermentation units, wherein the concentration of ethanol is intentionally increased to the elevated ethanol concentration in at least one of the fermentation units, and wherein the concentration of ethanol is untampered, or at least not intentionally increased to the elevated ethanol concentration, in at least one of the fermentation units.

Hereby, the different fermentation units can be adapted based on the target sugar (e.g. xylose or glucose) to ferment. For example, as described above with reference to various sub-steps of fermenting, in a fermentation unit configured to mainly ferment glucose (or any hexose), the concentration of ethanol is untampered, or at least not intentionally increased to the elevated ethanol concentration (it may e.g. be reduced) as this is advantageous for glucose fermentation, while in another fermentation unit configured to co-ferment xylose and glucose, the concentration of ethanol increased to the elevated ethanol concentration.

According to at least one example embodiment, a first fermentation unit in which the concentration of ethanol is intentionally increased to the elevated ethanol concentration is arranged downstream of a second fermentation unit in which the concentration of ethanol is untampered, or at least not intentionally increased to the elevated ethanol concentration (e.g. reduced).

The fermentation unit, or each fermentation unit, may comprise a vessel, i.e. a fermentation vessel.

According to at least one example embodiment, the initial amount of xylose relative the total amount of fermentable sugars in the fermentation media is at least 5 wt%.

The fermentable sugars may e.g. be sugars which are fermentable by Saccharomyces cerevisiae or another variant of Saccharomyces. The initial amount of xylose relative the total amount of fermentable sugars in the fermentation media may be at least 10 wt%, or at least 15 wt%, or at least 20 wt%.

According to at least one example embodiment, the step of intentionally increasing the concentration of ethanol to an elevated ethanol concentration comprises at least one of the following:
i) performing the step of fermenting in a continuous manner to in situ produce ethanol such that the elevated ethanol concentration is maintained during fermenting;
ii) external addition of ethanol, e.g. to be above 50 g/L;
iii) external addition of sugars producing ethanol such that the elevated ethanol concentration is maintained during fermenting;
iv) intentionally increasing the concentration of sugars in the fermentation media prior to, and/or during the step of fermenting, e.g. to be above 100 g/L, or above 140 g/L or above 180 g/L.

Hereby, efficient means for intentionally increasing the concentration of ethanol to an elevated ethanol concentration is provided. The external addition of ethanol according to option ii) may e.g. be above 70 g/L. The external addition of sugars producing ethanol according to option iii) may be above 100 g/L, or above 140 g/L. Typically, 100 g sugars/L results in the formation of approximately 50 g ethanol/L. The intentionally increasing the concentration of sugars according to option iv) may be carried out by removing liquid, such as water, in a dewatering step.

According to at least one example embodiment, the fermentation media further comprises mannose, and wherein the method further comprises the step of - controlling the temperature of the fermentation by a set temperature T < 28 °C.

In case of more than one fermentation unit, the control of temperature of the fermentation by a set temperature T < 28 °C, may be applied to at least one of the fermentation units.

According to at least one example embodiment, the yeast is a Saccharomyces sp. or an engineered variant thereof, e.g. a xylose fermenting engineered yeast.

A xylose fermenting engineered yeast is a yeast which has be engineered to ferment xylose. The yeast may be Saccharomyces cerevisiae or uvarum.

Thus, an engineered yeast, or a C5 yeast refers to a yeast configured to ferment pentoses (and hexoses), being any microorganism that is a member of the saccharomyces genus, and that has been engineered to be able to ferment one or more pentose sugars in addition to hexose sugars. Correspondingly, a non-engineered yeast, e.g. baker's yeast or a C6 yeast refers to a yeast configured to ferment hexoses (but not pentoses), thus being any microorganism that is a member of the saccharomyces genus, and that is naturally limited in fermentation ability to ferment only hexose sugars. For example, the C5 yeast is a yeast of the S. cerevisiae species that have been genetically engineered to be able to ferment one or more pentose sugars, such as xylose. Throughout the application the terms xylose fermenting yeast, C5 yeast, xylose fermenting engineered yeast or simply engineered yeast are used interchangeably, which is to be understood to be different from a non-xylose fermenting yeast, or a C6 yeast (i.e. a non-engineered yeast).

According to at least one example embodiment, the sugars are derived from hardwood, softwood, agricultural waste or any mixture thereof.

According to at least one example embodiment, the fermentation process is a continuous fermentation process, or is a batch fermentation process in which the yeast is recirculated, wherein the yeast is subject to regeneration, and wherein the elevated ethanol concentration is adapted to prevent, or at least reduce, oppression of xylose fermenting yeast.

Hereby, the fermentation of xylose may be carried out for a longer time. Thus, as the fermentation rate of xylose is increased relative to the fermentation rate of glucose owing to the elevated ethanol concentration, a regeneration of the yeast will result in less advantage for a yeast cell that has shed the xylose fermentation ability compared to a yeast cell that has the xylose fermentation ability maintained.

According to at least one example embodiment, the elevated ethanol concentration is at least 2 wt%, or at least 50 % of the theoretical yield of ethanol obtainable by the used fermentation media.

For example, the elevated ethanol concentration is at least 2.5 wt%, or 3 wt%, or 4 wt%, or 5 wt% or 6 wt% or 7 wt% or 8 wt%, or at least 10 wt %, with an upper limit of e.g. 20 wt%, such that the elevated ethanol concentration is between 6 wt% (or 8 wt % or 10 wt%) and 20 wt %. Alternatively, the elevated ethanol concentration is at least 50 % - 80 % of the theoretical yield of ethanol obtainable by the used fermentation media.

According to at least one example embodiment, the ethanol concentration is denoted X g/L, and the amount of yeast is denoted Y g/L, wherein the yeast stress ratio is defined as X/Y, wherein X/Y is at least 10.

According to at least one example embodiment, the elevated ethanol concentration is reached by the external addition of ethanol, or ethanol producing substance corresponding to the same amount of ethanol, of between 10 g/l to 50 g/l, such as e.g. between 10 g/l to 20g/l or 30 g/l, or 40 g/l, or between 20 g/l to 30 g/l or 40 g/l or 50 g/l.

According to at least one example embodiment, the elevated ethanol concentration is 25 % higher compared to what would normally be produced by the fermentable sugars in the fermentation media. For example, the elevated ethanol concentration is an ethanol concentration totaling above that typically achieved by fermenting the initially available fermentable sugars in the fermentation media at 25 wt% or higher sugar to ethanol conversion/yield. According to another example, the increased or elevated ethanol concentration is an ethanol concentration totaling between 6 and 9 wt%.

According to a second aspect of the present invention, a system for control of a microbial fermentation process involving fermentation of sugars from lignocellulosic biomass to fermentation products is provided. The system comprises:
- a fermentation unit configured to ferment a fermentation media comprising at least xylose and glucose by means of fermentation microorganisms of yeast; and
- means of intentionally increasing the concentration of ethanol present in the fermentation unit during the fermentation to an elevated ethanol concentration, wherein the means is configured to reach the elevated ethanol concentration by the addition of ethanol or an ethanol-producing substance.

Effects and features of this second aspect of the invention are largely analogous to those described above in connection with the first aspect of the invention. Embodiments mentioned in relation to the first aspect of the invention are largely compatible with the second aspect of the invention, of which some are exemplified below (typically without repeating the advantageous effects again).

According to at least one example embodiment, the system further comprises an ethanol determination arrangement configured to determine the ethanol concentration in the fermentation unit, and control means for automatically adjusting the ethanol concentration in the fermentation unit in a predetermined manner in response of determining that the concentration of ethanol is below a threshold limit.

The control means may e.g. be a control arrangement. The control arrangement may comprise adequate equipment, such as e.g. a controllable valve connected to a supply unit comprising ethanol or an ethanol-producing substance, which controllable valve is arranged on a supply line to, or upstream of, the fermentation unit, and is configured to vary the supply of ethanol or ethanol-producing substance in response of determining that the concentration of ethanol is below a threshold limit by means of the ethanol determination arrangement.

According to at least one example embodiment, the means of intentionally increasing the concentration of ethanol comprises at least one of the following:
a) means for operating the fermentation unit in a continuous manner to in situ produce ethanol such that the elevated ethanol concentration is maintained in the fermentation unit during the fermentation;
b) means for providing external addition of ethanol to, and/or upstream of, the fermentation unit;
c) means for providing external addition of sugars producing ethanol to, and/or upstream of the fermentation unit, such that the elevated ethanol concentration is maintained in the fermentation unit during the fermentation;
d) means for intentionally increasing the concentration of sugars in the fermentation media in, and/or upstream of the fermentation unit.

Thus, various options are conceivable for increasing the concentration of ethanol to the elevated ethanol concentration. For example, the means for operating the fermentation unit in a continuous manner may comprise an adequate fermentation unit, such as a continuous operable fermentation vessel. The means for providing external addition of ethanol may comprise a supply unit comprising the external ethanol, and a supply line for distributing the external ethanol to, and/or upstream of, the fermentation unit. Correspondingly, the means for providing external addition of sugars may comprise a supply unit comprising the external sugars, and a supply line for distributing the external sugars to, and/or upstream of, the fermentation unit. The supply unit may be configured to supply both external sugars and external ethanol, for example in two separated sub-compartments of the supply unit, and a respective (or single) supply line. The means for intentionally increasing the concentration of sugars in the fermentation may comprises a liquid removal arrangement, or a dewatering unit. Correspondingly, the control means or control arrangement may be configured to remove liquid in, or upstream of, the fermentation unit, e.g. for intentionally increasing the concentration of sugars in the fermentation media.

According to at least one example embodiment the system further comprises a temperature control arrangement configured to control the temperature of the fermentation unit during the fermentation by a set temperature T < 28 °C.

According to at least one example embodiment, the system and the fermentation unit is configured to carry out the fermentation as a continuous fermentation process, or as a batch fermentation process in which the yeast is recirculated, in which the yeast is subject to regeneration, and wherein the means of intentionally increasing the concentration of ethanol is adapted to prevent oppression of xylose fermenting yeast.

According to at least one example embodiment, the fermentation unit is a first fermentation unit and the system further comprises a second fermentation unit, the first and second fermentation units being configured to subsequently ferment the fermentation media, wherein the second fermentation unit is configured to ferment the fermentation media with an untampered ethanol concentration, or at least in the absence of intentionally increasing the ethanol concertation to an elevated ethanol concentration.

The fermentation unit(s) may be fermentation vessel(s) as previously described, and may be arranged as described in relation to the first aspect of the invention. That is, for example, the second fermentation unit may be arranged upstream of the first fermentation unit, and each one of the first and second fermentation units may according to an alternative embodiment be configured to ferment the fermentation media with an intentionally increased ethanol concentration to an elevated ethanol concentration. According to such embodiments, the system may comprise another fermentation unit being configured to ferment the fermentation media with an untampered ethanol concentration, or at least in the absence of intentionally increasing the ethanol concertation to an elevated ethanol concentration (such as e.g. a decreased ethanol concentration).

According to at least one example embodiment, the sugar concentration of the fermentation media is at least 5 g/L. According to at least one example embodiment, the fermentation media comprises galactose or alternatively galactose instead of glucose.

According to at least a third aspect of the invention, an arrangement for treatment of lignocellulosic biomass is provided. The arrangement comprises a pretreatment arrangement for pretreating the lignocellulosic biomass with e.g. SO2, a hydrolysis unit arranged downstream of and in fluid communication with the pretreatment arrangement, and a system for control of a microbial fermentation process comprising at least one fermentation unit, such as a fermentation vessel, arranged downstream of and in fluid communication with the hydrolysis unit. The system for control of a microbial fermentation process is typically the same as that described with reference to the second aspect of the invention.

Effects and features of this third aspect of the invention are largely analogous to those described above in connection with the second aspect of the invention. Embodiments mentioned in relation to the second aspect of the invention are largely compatible with at least the system for control of a microbial fermentation process of the third aspect of the invention.

For example, the arrangement may comprise additional units and components known to those skilled in the art. For example, a separation unit may be arranged between the pretreatment arrangement and the hydrolysis unit, and/or between the hydrolysis unit and the fermentation unit.

### BRIEF DESCRIPTION OF THE DRAWINGS

The various aspects of the present invention, including its particular features and advantages, will be readily understood from the following detailed description and the accompanying drawings, in which:
Fig. 1 is a graph showing the progress of the co-fermentation of a fermentation media including three sugars (glucose, mannose and xylose) by a non-xylose fermenting yeast;
Fig. 2 schematically illustrates an arrangement for treatment of lignocellulosic biomass including control of a microbial fermentation process in accordance with one example embodiment of the invention, and
Fig. 3 schematically illustrates a system for control of a microbial fermentation process in accordance with one example embodiment of the invention, and
Fig. 4 schematically illustrates the steps of a method for control of a microbial fermentation process in accordance with one example embodiment of the invention.
Fig. 5 is a graph showing the results of four measurement trials in accordance with one example embodiment of the invention; and
Fig. 6 is a graph showing the results of four measurement trials in accordance with one example embodiment of the invention.

### DETAILED DESCRIPTION

The present invention will now be described more fully hereinafter with reference to the accompanying drawings. The present invention may, however, be embodied in many different forms and should not be construed as limited to the embodiments set forth herein; rather, these embodiments are provided for thoroughness and completeness, and fully convey the scope of the present invention to the skilled person.

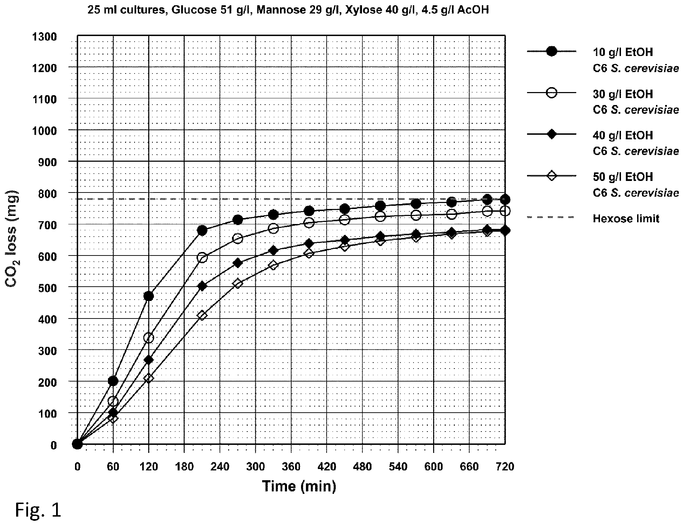

Fig. 1 is a graph showing the progress of the fermentation in terms of weight loss (mg) over time (min). The fermentation is here the simultaneous fermentation, or co-fermentation, of a fermentation media including three sugars (glucose, mannose and xylose) in a sugar mixture derived from the hemicellulose and cellulose of softwood, (Norway spruce). In the graph of Fig. 1, a non-xylose fermenting yeast (baker's yeast) was used for the same sugar mixture in four trials, the sugar mixtures being subject to various external addition of ethanol giving different elevated concentrations of ethanol (external addition of ethanol in grams per litre: 10 g/L, 30 g/L, 40 g/L and 50g/L). It is clear from Fig. 1 that fermentation of the hexoses (mannose and glucose) is reduced in the trial with 50 g/L added ethanol compared to the trial with only 10 g/L added ethanol. Thus, there is a clear correlation between increasing ethanol concentration and slower fermentation of the hexoses.

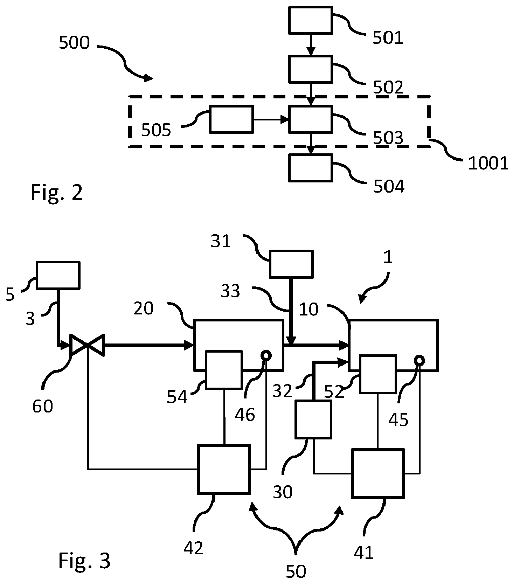

With reference to Fig. 2, the present disclosure provides an arrangement 500 for treatment of lignocellulosic biomass comprising a pretreatment arrangement 501 for pretreating the lignocellulosic biomass, a hydrolysis unit 502 in which the pretreated biomass is subject to enzymatic hydrolysis by means of saccharification enzymes, the hydrolysis unit being arranged downstream of and in fluid communication with the pretreatment arrangement 501, and comprises a fermentation unit 503, such as a fermentation vessel 503, arranged downstream of and in fluid communication with the hydrolysis unit 502. In the fermentation vessel 503, the hydrolysate is fermented into a target chemical, e.g. ethanol, by means of a yeast. The fermentation vessel 503 is comprised in a system 1001 configured for control of microbial fermentation, which may comprise additional units and components, such as e.g. a supply unit 505 connected to the fermentation vessel 503. The arrangement 500 may also comprise a product recovery unit 504, such as distillation or ion exchange chromatography, arranged downstream of and in fluid communication with the fermentation vessel 503. A system for control of a microbial fermentation process, as the system 1001, will now be described further with reference to Fig. 3.

Fig. 3 illustrates a system 1 for control of a microbial fermentation process involving fermentation of sugars from lignocellulosic biomass to fermentation products. The system 1 comprises a main fermentation unit 10 and a secondary fermentation unit 20. The secondary fermentation unit 20 is arranged upstream of the main fermentation unit 10. Each one of the main and secondary fermentation units may be a fermentation vessel 10, 20. Thus, the main and secondary fermentation units 10, 20 may constitute the fermentation unit 503 of Fig. 2. The main and secondary fermentation units 10, 20 are configured to ferment a fermentation media 3 comprising at least xylose and glucose by means of fermentation microorganisms of yeast. The fermentation media 3 may e.g. be fed to the system 1 by a hydrolysis unit as shown in Fig. 2. Thus, in operation, the main and secondary fermentation units 10, 20 comprises yeast and the fermentation media 3. As is clear from Fig. 3, the main fermentation unit 10 is arranged downstream of the secondary fermentation unit 20. For a continuous fermentation process as shown in Fig. 2, the fermentation media 3 will first be at least partly fermented in the secondary fermentation unit 20, where after the remaining fermentation media together with any produced fermented product, will subsequently be fermented in the main fermentation unit 10. Thus, the main fermentation unit 10 may be referred to as a first fermentation unit 10, or first fermentation vessel 10, and the secondary fermentation unit 20 may be referred to as a second fermentation unit 20, or second fermentation vessel 20.

The system 1 of Fig. 3 further comprises a moisture adjusting unit 5 in the form of a dewatering unit 5, configured to intentionally increase the concentration of sugars in the fermentation media 3 upstream of the main and secondary fermentation units 10, 20. The dewatering unit 5 may e.g. be arranged between the hydrolysis unit 503 and the main and secondary fermentation units 10, 20. Moreover, the system 1 comprises a supply unit 30 configured to supply external ethanol and/or an external ethanol-producing substance, such as external sugars, e.g. glucose, and a supply line 32 connecting the supply unit 30 with the main fermentation unit 10.

Thus, the supply unit 30 and supply line 32, as well as the dewatering unit 5, constitute different means of intentionally increasing the concentration of ethanol present in at least the main fermentation unit 10 during operation (i.e. during the fermentation) to an elevated ethanol concentration. For example, by increasing the concentration of sugars by means of the dewatering unit 5, an increased amount of ethanol will be produced in at least the secondary fermentation unit 20, which increased amount of ethanol will be transported together with the fermentation media to the main fermentation unit 10. According to another example, the concentration of sugars is increased such that both the main and secondary fermentation units 10, 20 are subject to the increased concentration of sugars, and the resulting increased concentration of ethanol. However, it should be noted that the system 1 needs not to be provided with both the dewatering unit 5 and the supply unit 30 with the supply line 32, but any means (including those mentioned below) for intentionally increasing the concentration of ethanol to an elevated ethanol concentration is sufficient. Thus, by the dewatering unit 5, and the supply unit 30 with the supple line 32, it is possible to reach the elevated ethanol concentration by the addition of ethanol or an ethanol-producing substance in at least the main fermentation unit 10.

Other means for intentionally increasing the ethanol concentration to the elevated ethanol concentration is by the addition of external ethanol or external sugars upstream of the main fermentation unit 10, in Fig. 3 embodied by a second supply unit 31 with corresponding second supply line 33 arranged between the main and secondary fermentation units 10, 20. A further option of intentionally increasing the ethanol concentration to the elevated ethanol concentration is by operating the main fermentation unit 10 in a continuous manner to in situ produce ethanol such that the elevated ethanol concentration is maintained in the main fermentation unit during the fermentation. Thus, by providing a fermentation media 3 with a predetermined sugar concentration which, during the fermentation in the main fermentation unit 10 will result in the production of ethanol, the elevated ethanol concentration may be reached.

The inventors have realized that the elevated ethanol concentration results in that the yeast ferment the xylose and glucose more equally compared to normal conditions, or conditions in which the ethanol concentration is not intentionally increased to the elevated ethanol concentration. Thus, by the elevated ethanol concentration, the fermentation of xylose relative the fermentation of glucose is increased. In other words, the relative fermentation rate of xylose to the fermentation rate of glucose is increased.

For example, the dewatering unit 5 is omitted, and the concentration of ethanol is untampered in the secondary fermentation unit 20, as described previously with reference to various sub-steps of fermenting. Moreover, the secondary fermentation unit 20 may be configured to ferment glucose but not xylose, e.g. by using a non-engineered yeast such as baker's yeast, while the main fermentation unit 10 is configured to co-ferment glucose and xylose in the presence of an ethanol concentration increased to the elevated ethanol concentration.

The operation of the main and secondary fermentation units 10, 20 are typically controlled by a respective first and second control unit 41, 42. Alternatively, a general control unit is used to control the operation of both the main and secondary fermentation unit 10, 20. For example, the second control unit 42 may be connected to a controllable valve 60 configured to control the flow of fermentation media 3 to the secondary fermentation unit 10 (or in embodiments in which the secondary fermentation unit 20 is omitted, to the main fermentation unit 10). The first control unit 41 may be configured to control the supply of external ethanol or external sugars from the supply unit 30 via the supply line 32 to the main fermentation unit 10. Moreover, the system 1 may comprise a first sensor 45 arranged to measure parameters in the main fermentation unit 10, and a second sensor 46 arranged to measure parameters in the secondary fermentation unit 20.

At least the first sensor 45 may be configured to determine the ethanol concentration in the main fermentation unit 10. Together with the first control unit 41 and the supply unit 30 with the supply line 32, the first sensor 45 may thus form an automatically ethanol adjusting arrangement configured to determine the ethanol concentration in the main fermentation unit 10 and automatically adjust the ethanol concentration in the main fermentation unit 10 by means of supply of external ethanol and/or external sugars. Additionally or alternatively, the first control unit 41 is connected to the dewatering unit 5 and may thus form an automatically ethanol adjusting arrangement together with the first sensor 45.

The system 1 may furthermore comprise a temperature control arrangement 50, here comprising the first sensor 45 acting as a first temperature sensor arranged to measure the temperature in the main fermentation unit 10 and a first temperature control element 52 configured to control the temperature of the main fermentation unit 10 (e.g. by means of a heating or cooling element). Correspondingly for the secondary fermentation unit 20, the temperature control arrangement 50 comprises the second sensor 46 acting as a second temperature sensor arranged to measure the temperature in the secondary fermentation unit 20 and a second temperature control element 54 configured to control the temperature of the secondary fermentation unit 20 (e.g. by means of a heating or cooling element). The first and second temperature control elements 52, 54 may be controlled by the first and second control units 41, 42, respectively. The first and second control units 41, 42 may e.g. be configured to control the temperature in the respective main and secondary fermentation units 10, 20 by a set temperature T < 28 °C.

It should be noted that the secondary fermentation unit 20 is optional and may be omitted, and that the fermentation unit may comprise a fermentation unit corresponding to the main fermentation unit 10 independent of the secondary fermentation unit 20.

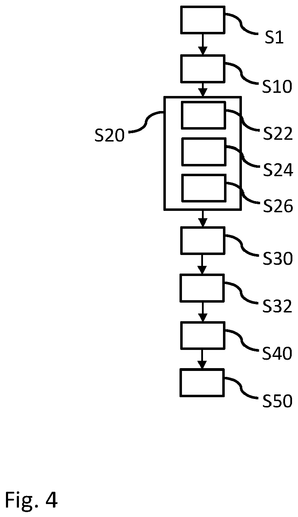

The invention will now be described with reference to the flow chart of Fig. 4 which e.g. includes the operation of the system 1 of Fig. 3. The flow chart of Fig. 4 discloses the steps of a method for control of a microbial fermentation process involving fermentation of sugars from lignocellulosic biomass to fermentation products,

In a step S1, a fermentation media comprising at least glucose and xylose is provided. The fermentation media and its sugars may be derived from hardwood, softwood, agricultural waste or any mixture thereof. The fermentation media may e.g. be supplied from a hydrolysis unit as shown in Fig. 2. The initial amount of xylose relative the total amount of fermentable sugars in the fermentation media may be at least 5 wt%.

In a step S10, the fermentation media is fermented by means of fermentation microorganisms of yeast. In Fig. 3, this is achieved by the main and secondary fermentation units 10, 20. The step S10 may comprises co-fermenting the xylose and glucose and/or comprise fermenting the xylose or glucose separately. According to one example embodiment, the step S10 is carried out in at least two separate fermentation units, wherein the concentration of ethanol is intentionally increased to the elevated ethanol concentration in at least one of the fermentation units, and the concentration of ethanol is untampered, or at least not intentionally increased to an elevated ethanol concentration, in at least one of the fermentation units. The yeast may e.g. be Saccharomyces or a variant thereof, e.g. a xylose fermenting engineered yeast.

In a step S20, the concentration of ethanol present during the fermentation is intentionally increased to an elevated ethanol concentration by the addition of ethanol or an ethanol-producing substance. Such intentional increase in the ethanol concentration to the elevated ethanol concentration may be carried out prior to and/or during the step of fermenting by e.g. utilizing means as described with reference to Fig. 3. The elevated ethanol concentration may e.g. be adapted such that the fermentation rate of xylose is above 20 % the fermentation rate of glucose, such as e.g. above 40 %, or above 60 % or above 80 % the fermentation rate of glucose. The elevated ethanol concentration may e.g. be at least 6 wt%, or at least 50 % of the theoretical yield of ethanol obtainable by the used fermentation media.

In various possible sub-steps, the step S20 may be carried out by:
Sub-step S22, by external addition of ethanol. The external ethanol may e.g. be added prior to, and/or during the step S10 to a final concentration of 50 g/L. In Fig. 3, the external addition of ethanol is achieved by the first and second supply units 30, 31 with corresponding supply lines 32, 33.
Sub-step S24, by external addition of sugars producing ethanol such that the elevated ethanol concentration is maintained during the step S10. In Fig. 3, the external addition of external sugars is achieved by the first and second supply units 30, 31 with corresponding supply lines 32, 33.
Sub-step S26, the concentration of sugars in the fermentation media is intentionally increased to be above 100 g/L, or above 140 g/L or above 180 g/L, prior to, and/or during the step of fermenting. In Fig. 3, this is achieved by the dewatering unit 5 prior to the secondary fermentation unit 20.

Alternatively, the step S20 is combined with the step S10 such that the step of fermenting, S10, is carried out in a continuous manner to in situ produce ethanol such that the elevated ethanol concentration is maintained, S20.

In a step S30, the ethanol concentration is determined (typically during the step S10), and in a step S32, the ethanol concentration is adjusted to be above a threshold limit in response of determining that the concentration of ethanol is below the threshold limit. The threshold limit may e.g. be based on the relation of fermentation rates of glucose and xylose. For example, in the system 1 of Fig. 3, the first sensor 45 is used together with the first control unit 41 to instruct the supply unit 30 to supply a predetermined amount of ethanol or ethanol-producing substance (e.g. external sugars) via the supply line 32 to the main fermentation unit 10.

In a step S40, typically carried out together with the step S10, the temperature of the fermentation is controlled by a set temperature T < 28 °C. For such embodiments, the fermentation media typically comprises mannose.

The system 1 in Fig. 3 is configured to carry out the fermentation as a continuous fermentation process in the main and secondary fermentation units 10, 20, but it may as well be configured as a batch fermentation process in which the yeast is recirculated. In both these options, the yeast is subject to regeneration, and the means of intentionally increasing the concentration of ethanol may be adapted to prevent oppression of xylose fermenting yeast. Thus, the method may comprise a step S50, e.g. comprised in the step S20, or performed instead of step S20, of adapting the elevated ethanol concentration to prevent oppression of xylose fermenting yeast.

For embodiments in which the method is automated and adapted for continuous operation, the method may, alternatively or in addition to the ethanol concertation and the temperature during the fermenting, comprise a step of collecting and monitoring various process parameters in a continuous or semi-continuous manner and a step of automatically adjusting the fermenting process in response to the monitored process parameters.

### EXAMPLES

Co-fermentation of a fermentation media including three sugars (glucose, mannose and xylose) in a sugar mixture derived from the hemicellulose and cellulose of softwood, (Norway spruce) was performed batch-wise with 25 ml cultures in 50 ml bottles, consisted of diluted spruce hydrolysate containing 51 g/l glucose, 29 g/l mannose and fortified with added xylose to a resultant concentration of 40 g/l xylose. Acetic acid was adjusted to 4.5 g/l to reflect the content in undiluted hydrolysate, pH was adjusted to 5.5 with ammonia, and 3 g/l urea was added to ensure sufficient nitrogen. Four measurement trials, trial 1-4, were carried out for the sugar mixture with various external addition of ethanol giving different elevated concentrations of ethanol (external addition of ethanol in grams per litre: 10 g/L, 30 g/L, 40 g/L and 50g/L). For all trials, a xylose fermenting engineered yeast was used, using an inoculation amount of 40 g DW yeast/l, a temperature of 30 °C, and shaking on an orbital shaker set at 150 rpm.

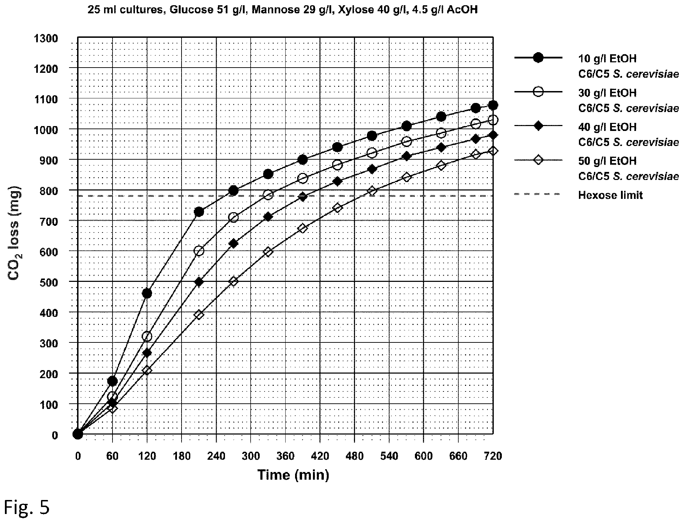

The results are presented in the graph of Fig. 5 showing the progress of the fermentation in terms of weight loss (represented by CO2 loss in mg) over time (min). The fermentation limit of the hexoses (i.e. glucose and mannose) are shown as a vertical dotted line. It is clear from Fig. 5 that the fermentation rates of the three sugars are more equal at a higher ethanol concentration due to the fact that the xylose fermentation rate (seen above the hexose limit line) is almost unaffected by the ethanol concentration (the curves are almost parallel here), whereas the glucose rate at the 50 g/1 added ethanol is about 40 % of the rate with 10 g/l added ethanol, comparing the rates between 60 and 120 minutes. Note that the in situ generated ethanol should be considered, so that the ethanol concentration at the 780 mg hexose limit is 35 g/l in situ generated ethanol in addition to the externally added ethanol. Thus, at a high ethanol concentration, the yeast ferments all three sugars with a more equal efficiency. This is e.g. noticeable by the lack of the "knee-bend" or rather lack of the sudden reduced slope of the curve that occurs in e.g. Fig. 1 between 180 and 300 minutes for the corresponding trials with 50 g ethanol/l.

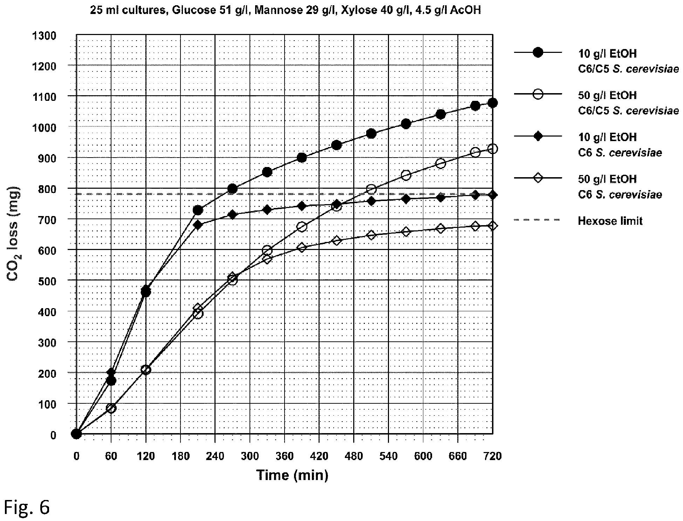

Fig. 6 is a graph showing the results of trials 1 and 4 (i.e. external addition of ethanol with 10 g/L and 50 g/L, respectively) from Fig. 5, together with two trials carried out for the same sugar mixture and as described above with the only difference that the yeast is not capable to ferment xylose (i.e. a non-engineered yeast, e.g. baker's yeast) for external addition of ethanol with 10 g/L and 50 g/L, respectively. Thus, Fig. 6 discloses a pairwise comparison of a xylose fermenting and a non-xylose fermenting yeasts, at the lowest and highest external ethanol additions.

From Fig. 6 it is clear the xylose fermenting ability of the yeast, also increases the ability to ferment the hexoses near the hexose limit, and that at an external addition of ethanol of 50 g/l there is no shift caused by different fermentation rates of hexose and xylose as the curve passes the hexose limit. This is believed to be because the xylose fermentation rate is unaffected by the ethanol addition, whereas the hexose fermentation rate is reduced to, or towards, the same level as the xylose fermentation rate.

Moreover, by comparing the trials with the different yeasts, and the respective high (50 g/L) and low (10g/L) external ethanol addition, it is clear that the xylose fermenting engineered yeast has an advantage in fermentation rate and thus growth rate also in the phase where hexoses still remains to be fermented. This is likely due to the fact that the fermentation rate of xylose in the presence of higher ethanol concentrations does not slow down as the fermentation approaches the hexose limit. In other words, a yeast which is incapable of fermenting xylose (e.g. a yeast which has shed the xylose fermentation ability is at growth rate disadvantage already in the late hexose phase. Thus, the elevated ethanol concentration results in a higher stability of the xylose fermenting yeast, in for example a continuous fermentation process, even when running it continuously at conditions where hexose is still present.

As indicated in Figs. 5 and 6, the ethanol concentration should preferably reach a certain threshold level or elevated ethanol concentration for the fermentation rates of xylose and glucose to be equal, or substantially equal (e.g. that the fermentation rate of xylose is within 70 % of the fermentation rate of glucose) and thus keeping the transmembrane transporters induced until xylose is exhausted. Without being bound by any theory, the inventors believe that this is caused by a reduction of transmembrane transporter affinity towards glucose, until glucose and xylose is taken up by the yeast at the same rate, and thus balancing the co-fermentation of xylose and glucose, keeping the glucose present and transmembrane transporters induced until both xylose and glucose have been exhausted, effectively enabling a faster and more complete xylose fermentation throughout at least a part of the fermentation process.

Even though the present disclosure has been described with reference to specific exemplifying embodiments thereof, many different alterations, modifications and the like will become apparent for those skilled in the art.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

## Claims

1. A method for control of a microbial fermentation process involving fermentation of sugars from lignocellulosic biomass to fermentation products, comprising the steps of:
- providing a fermentation media comprising at least xylose and glucose;
- fermenting the fermentation media by means of fermentation microorganisms of yeast; and
- intentionally increasing the concentration of ethanol present during the fermentation to an elevated ethanol concentration by the addition of ethanol or an ethanol-producing substance.

2. The method according to claim 1, wherein the elevated ethanol concentration is adapted such that the fermentation rate of xylose is within 20 % the fermentation rate of glucose, such as e.g. within 40 %, or within 60 % or within 80 % the fermentation rate of glucose.

3. The method according to any one of the preceding claims, wherein the step of fermenting comprises co-fermenting the xylose and glucose.

4. The method according to any one of the preceding claims, further comprising the steps of:
- determining the ethanol concentration during the step of fermenting,
- in response of determining that the concentration of ethanol is below a threshold limit, adjusting the ethanol concentration to be above the threshold limit.

5. The method according to claim 4, wherein the threshold limit is based on the relation or ratio of fermentation rates of glucose and xylose.

6. The method according to any one of the preceding claims, wherein the step of fermenting is carried out in at least two separate fermentation units, and wherein the concentration of ethanol is intentionally increased to the elevated ethanol concentration in at least one of the fermentation units, and wherein the concentration of ethanol is untampered, or at least not intentionally increased to an elevated ethanol concentration, in at least one of the fermentation units.

7. The method according to any one of the preceding claims, wherein the initial amount of xylose relative the total amount of fermentable sugars in the fermentation media is at least 5 wt%.

8. The method according to any one of the preceding claims, wherein the step of intentionally increasing the concentration of ethanol to an elevated ethanol concentration comprises at least one of the following:
i) performing the step of fermenting in a continuous manner to in situ produce ethanol such that the elevated ethanol concentration is maintained during fermenting;
ii) external addition of ethanol, e.g. to be above 50 g/L;
iii) external addition of sugars being converted into ethanol such that the elevated ethanol concentration is maintained during fermenting;
iv) intentionally increasing the concentration of sugars in the fermentation media prior to, and/or during the step of fermenting, e.g. to be above 100 g/L, or above 140 g/L or above 180 g/L.

9. The method according to any one of the preceding claims, wherein the fermentation media further comprises mannose, and wherein the method further comprises the step of
- controlling the temperature of the fermentation by a set temperature T < 28 °C.

10. The method according to any one of the preceding claims, wherein the yeast is a Saccharomyces sp. or a variant thereof, e.g. a xylose fermenting engineered yeast.

11. The method according to any one of the preceding claims, wherein the sugars are derived from hardwood, softwood, agricultural waste or any mixture thereof.

12. The method according to any one of the preceding claims, wherein the fermentation process is a continuous fermentation process, or is a batch fermentation process in which the yeast is recirculated, wherein the yeast is subject to regeneration, and wherein the elevated ethanol concentration is adapted to prevent oppression of xylose fermenting yeast.

13. The method according to any one of the preceding claims, wherein the elevated ethanol concentration is at least 2 wt%, or at least 50 % of the theoretical yield of ethanol obtainable by the used fermentation media.

14. A system for control of a microbial fermentation process involving fermentation of sugars from lignocellulosic biomass to fermentation products, the system comprising:
- a fermentation unit configured to ferment a fermentation media comprising at least xylose and glucose by means of fermentation microorganisms of yeast; and
- means of intentionally increasing the concentration of ethanol present in the fermentation unit during the fermentation to an elevated ethanol concentration, wherein the means is configured to reach the elevated ethanol concentration by the addition of ethanol or an ethanol-producing substance.

15. The system according to claim 14, further comprising an ethanol determining arrangement configured to determine the ethanol concentration in the fermentation unit, and control means for automatically adjusting the ethanol concentration in the fermentation unit in a predetermined manner in response of determining that the concentration of ethanol is below a threshold limit.

16. The system according to any one of claims 14-15, wherein the means of intentionally increasing the concentration of ethanol comprises at least one of the following:
a) means for operating the fermentation unit in a continuous manner to in situ produce ethanol such that the elevated ethanol concentration is maintained in the fermentation unit during the fermentation;
b) means for providing external addition of ethanol to, and/or upstream of, the fermentation unit;
c) means for providing external addition of sugars being convertible into ethanol to, and/or upstream of the fermentation unit, such that the elevated ethanol concentration is maintained in the fermentation unit during the fermentation;
d) means for intentionally increasing the concentration of sugars in the fermentation media in, and/or upstream of the fermentation unit.

17. The system according to any one of claims 14-16, further comprising a temperature control arrangement configured to control the temperature of the fermentation unit during the fermentation by a set temperature T < 28 °C.

18. The system according to any one of claims 14-17, wherein the system and the fermentation unit is configured to carry out the fermentation as a continuous fermentation process, or as a batch fermentation process in which the yeast is recirculated, in which the yeast is subject to regeneration, and wherein the means of intentionally increasing the concentration of ethanol is adapted to prevent oppression of xylose fermenting yeast.

19. The system according to any one of claims 14-18, wherein the fermentation unit is a first fermentation unit and the system further comprises a second fermentation unit, the first and second fermentation units being configured to subsequently ferment the fermentation media, wherein the second fermentation unit is configured to ferment the fermentation media with an untampered ethanol concentration, or at least in the absence of intentionally increasing the ethanol concertation to an elevated ethanol concentration.
